# FASCICULE DE BREVET EUROPEEN

(11) **EP 4 048 820 B1**
(45) Date de publication et mention de la délivrance du brevet: **10.04.2024**
(21) Numéro de dépôt: 20792267.5
(22) Date de dépôt: 14.10.2020
(51) Int. Cl.: C12R 1/01, A23L 33/135, A23C 9/12

(54) **NOUVELLES SOUCHES DE CARNOBACTERIUM MALTAROMATICUM ET LEURS UTILISATIONS**
NEUE STÄMME VON CARNOBACTERIUM MALTAROMATICUM UND IHRE VERWENDUNGEN
NEW STRAINS OF CARNOBACTERIUM MALTAROMATICUM AND THEIR USES

(30) Priorité: 24.10.2019 FR 1911895
(43) Date de publication de la demande: 31.08.2022
(73) Titulaire: Université de Lorraine, 54000 Nancy (FR)
(72) Inventeur: BORGES, Frédéric, 54230 CHAVIGNY (FR); REVOL-JUNELLES, Anne-Marie, 54600 VILLERS LES NANCY (FR)
(74) Mandataire: IPAZ
(86) Numéro de dépôt international: PCT/EP2020/078966
(87) Numéro de publication internationale: WO 2021/078612

(56) Documents cités:
- AFZAL MUHAMMAD INAM ET AL: "Characterization ofCarnobacterium maltaromaticumLMA 28 for its positive technological role in soft cheese making", FOOD MICROBIOLOGY, ACADEMIC PRESS LTD, LONDON, GB, vol. 36, no. 2, 12 juin 2013 (2013-06-12), pages 223-230, XP028708731, ISSN: 0740-0020, DOI: 10.1016/J.FM.2013.05.008
- VYTRASOVA J ET AL: "Influence of the preservative material HOLDBAC< TM > on the growth and proliferation of Listeria on the surfaces of cheeses", CZECH JOURNAL OF FOOD SCIENCE, PRAGUE, CZ , vol. 28, no. 3 1 janvier 2010 (2010-01-01), pages 242-248, XP008153566, ISSN: 1212-1800 Extrait de l'Internet: URL:http://www.agriculturejournals.cz/publ icFiles/22810.pdf [extrait le 2020-06-08]
- C. Caillez-Grimal ET AL: "Short Communication: Carnobacterium maltaromaticum:The OnlyCarnobacteriumSpecies in French Ripened Soft Cheesesas Revealed by Polymerase Chain Reaction Detection", J. Dairy Sci. 90, 1 janvier 2007 (2007-01-01), pages 1133-1138, XP055702337, Extrait de l'Internet: URL:https://doi.org/10.3168/jds.S0022-0302 (07)71599-0 [extrait le 2020-06-08]

## Description

La présente demande concerne des souches de *Carnobacterium maltaromaticum,* une préparation bactérienne comprenant au moins une souche de *Carnobacterium maltaromaticum,* et leur utilisation dans la préparation d'un produit alimentaire.

La présence de bactéries pathogènes dans les aliments est une problématique de santé publique majeure. Dans le secteur de la fromagerie, la présence potentielle de *Listeria monocytogenes* est une préoccupation constante. Sa présence dans les produits laitiers transformés fait l'objet d'incidents mettant en jeu la santé du consommateur et la stabilité économique des acteurs de la filière. A titre d'exemple, on peut citer le retrait de la commercialisation de plusieurs lots de fromage contaminés par *Listeria monocytogenes* fortement médiatisé en décembre 2015. En conséquence, les acteurs de la filière sont activement à la recherche de solutions permettant de mieux maîtriser le danger *Listeria.* Certaines sociétés spécialisées dans la production et la commercialisation de micro-organismes proposent des cultures microbiennes biopréservatrices présentant des propriétés *anti-Listeria.* Par exemple, il est connu que certaines souches de *Lactobacillus plantarum* possèdent une activité *anti-Listeria.* Une souche de *Lactobacillus plantarum* est déjà commercialisée sous la marque HOLDBAC^{®} en tant que ferment de bio-préservation.

Toutefois, malgré l'utilisation de ces cultures biopréservatrices, la présence de *Listeria monocytogenes* subsiste, très probablement parce que les cultures biopréservatrices proposées sont des bactéries lactiques classiques ne permettant pas d'apporter une protection optimale et durable tout au long de la vie du produit alimentaire. D'ailleurs, tous les industriels n'utilisent pas des procédés de ce type car les solutions proposées ne sont pas compatibles avec tous les procédés de fabrications et tous les types de fromages.

L'activité *anti-Listeria* de souches de bactéries lactiques de l'espèce *Carnobacterium maltaromaticum* a déjà été décrite dans l'art antérieur. Par exemple, Afzal et al. (Food Microbiology 36(2013) 223-230) décrivent une souche de *Carnobacterium maltaromaticum* LMA28 qui présente une activité *anti-Listeria monocytogenes.*

Cependant, jusqu'à présent, les bactéries biopréservatrices existantes sur le marché ainsi que les souches de *Carnobacterium maltaromaticum* décrites dans la littérature sont toutes identifiées et sélectionnées en utilisant un critère de sélection basé uniquement sur la capacité inhibitrice de *Carnobacterium maltaromaticum* vis-à-vis d'une seule souche de *L. monocytogenes.*

Les souches sélectionnées selon ce critère ne peuvent pas satisfaire aux besoins et exigences rencontrés dans l'industrie alimentaire, notamment dans la production de fromage.

Les Inventeurs de la présente invention ont indiqué pour la première fois l'importance de la robustesse dans l'efficacité anti-bactérienne pour une souche de *Carnobacterium maltaromaticum* et mis en place trois critères qui permettent d'isoler et sélectionner des souches qui possèdent une robustesse importante.

Le terme « robustesse » est défini ici par une bactérie dont l'activité antibactérienne est maintenue (1) même si elle est ensemencée à faible concentration, (2) même si elle est ensemencée tardivement par rapport à *Listeria monocytogenes* et (3) si elle inhibe une grande diversité de souches de *Listeria monocytogenes.*

A ce jour, aucune souche de *Carnobacterium maltaromaticum* décrite dans l'art antérieur ne satisfait à l'ensemble de ces critères. Il est par conséquent nécessaire de sélectionner de nouvelles souches de *Carnobacterium maltaromaticum* pour son utilisation à des fins de biopréservation d'un produit alimentaire.

La présente invention a pour objet de proposer de nouvelles souches de *Carnobacterium maltaromaticum.*

Afin qu'une souche de *Carnobacterium maltaromaticum* puisse être retenue et considérée comme possédant une robustesse satisfaisante, trois critères de sélection sont préétablis. Trois critères sont appliqués simultanément pour le criblage de souches :
- une efficacité *anti-Listeria* avec un faible taux d'ensemencement, à savoir inférieur à 10² UFC.mL⁻¹ de *Carnobacterium maltaromaticum* initial
- une efficacité *anti-Listeria* même quand il y a un ensemencement tardif de *Carnobacterium maltaromaticum* par rapport à celui de *Listeria,* même en faible taux d'ensemencement,
- un spectre d'activité vis-à-vis de diverses souches de *Listeria monocytogenes* et de *Listeria* large.

Du fait que les collections de *Carnobacterium maltaromaticum* sont sélectionnées sur la base de l'activité d'une souche de *Carnobacterium maltaromaticum* contre une souche spécifique de *Listeria monocytogenes,* il est impossible de prédire qu'une souche répondant à l'ensemble des trois critères puisse exister. Contre toute attente, deux souches de *Carnobacterium maltaromaticum* ont été isolées à l'issue de plusieurs campagnes de criblage de *Carnobacterium maltaromaticum* en appliquant simultanément les trois critères ci-dessus.

Aussi l'invention a pour objet une souche CNCM I-5242 déposée le 26 septembre 2017 et une souche CNCM I-5243 déposée le 26 septembre 2017 auprès de la Collection Nationale de Cultures de Microorganismes.

Ces deux souches présentent une activité *anti-Listeria monocytogenes* supérieure à celle de la souche de *Lactobacillus plantarum* commercialisée sous la marque HOLDBAC^{®} (No. de produit 1209325) en tant que souche de bio préservation.

Même à un taux d'ensemencement inférieur à 100 UFC.mL⁻¹, ces deux souches isolées montrent une activité anti-*Listeria* efficace.

Par ailleurs, ces deux souches présentent une activité *anti-Listeria* significative vis-à-vis de la plupart de souches de *Listeria monocytogenes* testées pendant les expériences de criblage.

Même en co-culture, c'est-à-dire lors de l'ensemencement simultané avec des *Listeria monocytogenes,* condition qui correspond à celle que l'on peut rencontrer réellement en fabrication fromagère, ces deux souches peuvent encore inhiber significativement la croissance de *Listeria monocytogenes* dans la co-culture.

En plus, les tests de fabrication des fromages montrent que ces souches n'inhibent pas la croissance des ferments lactiques et des ferments d'affinage. L'utilisation de ces souches peut même apporter aux fromages un arôme particulier et agréable, dans certaines conditions et types de fabrication.

Un autre aspect de la présente invention concerne une préparation bactérienne comprenant la souche CNCM I-5242 ou la souche CNCM I-5243.

On entend par « préparation bactérienne » une composition conditionnée pour la croissance de *Carnobacterium maltaromaticum,* ladite composition comprenant une souche de *Carnobacterium maltaromaticum* viable et des composants essentiels pour la croissance des bactéries, tels qu'une source de carbone et d'énergie, une source de potassium et de phosphore, une source de calcium, une source de magnésium, des lipides, des protéines ou des acides aminés.

Ladite préparation bactérienne peut comprendre un milieu de culture ou un extrait de milieu de culture. Ledit milieu peut être le milieu TSB-YE (Bouillon Tryptone Soja-Extrait de Levure).

Ledit milieu de culture peut être un milieu synthétique. Par milieu synthétique, on entend selon la présente invention un milieu dans lequel sont introduits des composants individuels caractérisés et soumis à un contrôle quantitatif et qualitatif rigoureux.

Ledit milieu de culture peut être aussi un milieu naturel. Par milieu naturel, on entend selon la présente invention un milieu qui contient un ou plusieurs composés naturels dans sa composition. Ledit milieu naturel peut être du lait, notamment du lait entier pasteurisé.

Ladite préparation bactérienne peut être sous forme liquide ou solide.

Dans un mode de réalisation de l'invention, la concentration de *Carnobacterium maltaromaticum* dans ladite préparation bactérienne de l'invention peut être de 10² UFC.mL⁻¹ à 10¹¹ UFC.mL⁻¹, notamment de 10⁵ UFC.mL⁻¹ à 10⁷ UFC.mL⁻¹, plus particulièrement d'au moins 10⁵ UFC.mL⁻¹, ou d'au moins 10⁶ UFC.mL⁻¹, ou d'au moins 10⁷ UFC.mL⁻¹, par rapport au volume total de la préparation bactérienne.

Dans un autre mode de réalisation de l'invention, ladite préparation bactérienne contient une quantité de *Carnobacterium maltaromaticum* de 10² UFC.g⁻¹ à 10¹¹ UFC.g⁻¹, notamment de 10⁵ UFC.g⁻¹ à 10⁷ UFC.g⁻¹, d'au moins 10⁵ UFC.g⁻¹, ou d'au moins 10⁶ UFC.g⁻¹, ou d'au moins 10⁷ UFC.g⁻¹, par rapport au poids total de la préparation bactérienne.

Dans un mode de réalisation particulier, ladite préparation bactérienne est un concentrât liquide, un concentrât congelé, ou un lyophilisat.

On entend par « concentrât » un liquide contenant une quantité de bactérie supérieure à 10⁵ UFC.mL⁻¹, en particulier supérieure à 10¹⁰ UFC.mL⁻¹, notamment supérieure à 10¹¹ UFC.mL⁻¹, par rapport au volume total du concentrât.

Le concentrât peut être obtenu par toute méthode conventionnelle, par exemple par centrifugation ou par microfiltration d'une culture de *Carnobacterium maltaromaticum* après l'incubation adaptée.

On entend par « lyophilisat » le produit obtenu après lyophilisation d'une culture de *Carnobacterium maltaromaticum* après l'incubation adaptée.

On entend par « incubation adaptée » une incubation selon une méthode conventionnelle pour obtenir une population initiale de *Carnobacterium maltaromaticum* proche de 10⁷ UFC.mL⁻¹, par rapport au volume total de l'incubation.

A titre d'exemple, l'incubation de *Carnobacterium maltaromaticum* peut être effectuée à 30°C pendant 48 heures dans du lait entier pasteurisé.

Dans un mode de réalisation particulier, ladite préparation bactérienne est un concentrât, éventuellement congelé, comprenant du lait entier pasteurisé et d'au moins 10⁷ UFC.mL⁻¹ de *Carnobacterium maltaromaticum* CNCM I-5242, par rapport au volume total de la préparation bactérienne.

Dans un autre mode de réalisation particulier, ladite préparation bactérienne est un concentrât, éventuellement congelé, comprenant du lait entier pasteurisé et d'au moins 10⁷ UFC.mL⁻¹ de *Carnobacterium maltaromaticum* CNCM I-5243, par rapport au volume total de la préparation bactérienne.

Dans un mode de réalisation de l'invention, ladite préparation bactérienne peut comprendre en outre d'au moins un ferment lactique et/ou au moins un ferment d'affinage.

Selon un mode de réalisation de l'invention, ladite préparation bactérienne est une culture protectrice de *Carnobacterium maltaromaticum.*

On entend par « culture protectrice de *Carnobacterium maltaromaticum »* une composition contenant *Carnobacterium maltaromaticum* vivantes qui peut être ajoutée dans les produits alimentaires pour inhiber la croissance des microorganismes pathogènes ou toxiques pour l'homme ou l'animal et présents dans lesdits produits alimentaires, afin de réduire le risque de contamination de produit alimentaire par ces microorganismes pathogènes ou toxiques.

Au sens de la présente invention, ledit microorganisme pathogène ou toxique est une bactérie du genre *Listeria,* notamment *Listeria monocytogenes, Listeria innocua, Listeria ivanovii.*

Ladite culture protectrice peut être sous forme de concentrât liquide ou sous forme de lyophilisat.

La présente invention concerne aussi l'utilisation de *Carnobacterium maltaromaticum,* de la souche CNCM I-5242 ou de la souche CNCM I-5243, ou une préparation bactérienne telle que décrite ci-dessus, ou une culture protectrice telle que décrite ci-dessus, dans la préparation d'un produit alimentaire.

Plus particulièrement, les souches de *Carnobacterium maltaromaticum,* la préparation bactérienne ou la culture protectrice décrite ci-dessus sont utilisées pour inhiber la croissance des bactéries du genre *Listeria* dans un produit alimentaire.

Ladite culture protectrice de l'invention peut être utilisée dans la production des produits laitiers fermentés ou non fermentés, notamment dans la production de fromages ou de laits fermentés.

Dans un mode de réalisation particulier, ledit produit alimentaire est un produit laitier fermenté, notamment les fromages, tels que les fromages à pâte molle, fromages à pâte pressée cuites ou non cuite, fromages frais, fromages de chèvre, fromages de brebis, ou des laits fermentés.

Ladite culture protectrice de l'invention peut être aussi utilisée dans la production des autres produits laitiers, tels la crème, la crème glacée, le beurre, ou les produits secondaires dérivés du lait, comme le lactosérum, la caséine, ou d'autres produits alimentaires préparés contenant comme ingrédient du lait ou des constituants du lait.

Ladite culture protectrice de l'invention peut être aussi utilisée dans la production de boisson, par exemple les jus de fruits, les boissons obtenues avec les mélanges de lait et de jus de fruits, le lait de soja, le lait d'avoine, le lait de riz ou de produits végétaux fermentés.

Ladite culture protectrice de l'invention peut être aussi utilisée dans la production industrielle de produits de salaisons et charcuterie.

L'utilisation de la culture protectrice de l'invention pendant la fabrication des produits alimentaires permet d'inhiber la croissance des bactéries du genre *Listeria* qui subsistent dans un produit alimentaire en faible quantité et de prolonger la période de conservation dudit produit alimentaire.

La culture protectrice de *Carnobacterium maltaromaticum* selon la présente invention est ajoutée dans les produits alimentaires selon le type de produit.

Pour les produits laitiers fermentés, tels que les fromages ou les laits fermentés, la culture protectrice est ajoutée avant ou en même temps que les ferments lactiques ou après leur addition.

Pour les autres produits alimentaires, la culture protectrice peut être ajoutée en fin de ligne de production et notamment avant le conditionnement du produit alimentaire.

A titre d'exemple, la culture protectrice de *Carnobacterium maltaromaticum* selon la présente invention est additionnée dans un produit alimentaire liquide pour atteindre un niveau de 10² UFC.mL⁻¹ à 10¹¹ UFC.mL⁻¹, notamment de 10⁵ UFC.mL⁻¹ à 10⁷ UFC.mL⁻¹, plus particulièrement d'au moins 10⁶ UFC.mL⁻¹, notamment entre 10⁶ UFC.mL⁻¹ et 10⁷ UFC.mL⁻¹, par rapport au volume total dudit produit alimentaire liquide ; ou additionnée dans un produit alimentaire solide pour atteindre un niveau de 10² UFC.g⁻¹ à 10¹¹ UFC.g⁻¹, notamment de 10⁵ UFC.g⁻¹ à 10⁷ UFC.g⁻¹, d'au moins 10⁵ UFC.g⁻¹, ou d'au moins 10⁶ UFC.g⁻¹, ou d'au moins 10⁷ UFC.g⁻¹, par rapport au poids total dudit produit alimentaire solide, pendant la période de conservation dudit produit alimentaire.

Un autre aspect de l'invention concerne un procédé de préparation d'un fromage, notamment un fromage à pâte molle ou un fromage à pâte pressée non cuite, utilisant la souche CNCM I-5242 ou la souche CNCM I-5243 ou une préparation bactérienne contenant l'une des deux souches.

Ledit procédé comprend :
- l'ensemencement par des bactéries *Carnobacterium maltaromaticum* souche CNCM I-5242 ou CNCM I-5243 ou une préparation bactérienne contenant la souche CNCM I-5242 ou CNCM I-5243 dans le lait ou pendant la transformation du lait.

Selon le procédé de l'invention, l'ensemencement par des bactéries *Carnobacterium maltaromaticum* peut être effectué dans le lait, dans le lactosérum, dans la saumure, ou directement sur la pâte de fromage.

Selon le procédé de l'invention, lorsque l'ensemencement est fait sur la pâte de fromage, il peut être effectué par pulvérisation ou par spray d'une préparation liquide de *Carnobacterium maltaromaticum* sur la pâte de fromage.

On entend par « ensemencement pendant la transformation du lait » dans la fabrication du fromage, l'ensemencement pendant le stockage, la coagulation, l'égouttage, le moulage, le pressage, le salage, l'affinage, ou le lavage.

Selon le procédé de l'invention, lorsque l'ensemencement est fait dans le lait, l'ensemencement peut être effectué avec ou sans maturation froide du lait.

Par ailleurs, l'ensemencement dans le lait par des bactéries *Carnobacterium maltaromaticum* de l'invention peut être effectué avant ou simultanément avec l'ensemencement des ferments lactiques.

Dans un autre mode de réalisation, l'ensemencement de *Carnobacterium maltaromaticum* est effectué de 0 à 8 heures avant celui des ferments lactiques.

Le lait utilisé dans ledit procédé peut être du lait, entier ou non, cru ou pasteurisé ou ayant subi un traitement thermique.

On entend par « lait entier pasteurisé » du lait entier ayant subi un traitement thermique à 71,5°C pendant 15 secondes, puis rapidement refroidi à 4°C.

Dans un mode de réalisation du procédé, le lait est ensemencé par *Carnobacterium maltaromaticum* à un taux de 10² UFC.mL⁻¹ à 10¹¹ UFC.mL⁻¹, notamment de 10⁶ UFC.mL⁻¹ et 10⁷ UFC.mL⁻¹, par rapport au volume du lait.

La présente invention concerne également un procédé pour réduire la contamination microbienne lors du processus de fabrication d'un produit laitier fermenté comprenant l'addition d'une quantité inhibitrice de *Carnobacterium maltaromaticum* souche CNCM I-5242 ou CNCM I-5243 ou une préparation bactérienne contenant la souche CNCM I-5242 ou CNCM I-5243.

La présente invention propose également un procédé pour réduire la contamination par des bactéries du genre *Listeria* lors du processus de fabrication d'un produit laitier fermenté caractérisé en ce qu'il comprend l'addition d'une quantité de 10² UFC.mL⁻¹ à 10¹¹ UFC.mL⁻¹, notamment de 10⁶ à 10⁷ ufc.mL⁻¹, par rapport au volume du produit laitier, de *Carnobacterium maltaromaticum* souche CNCM I-5242 ou CNCM I-5243 ou une préparation bactérienne comprenant ces souches.

Les différents aspects de l'invention sont présentés plus en détail ci-dessous par les figures et les exemples.

### Figures

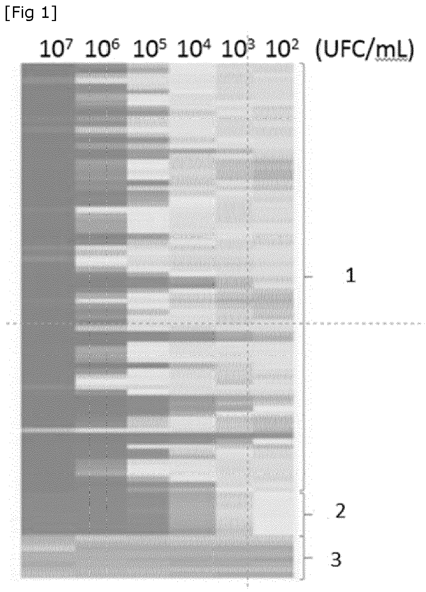

[Fig 1] Influence du taux d'ensemencement sur les propriétés *anti-Listeria monocytogenes* d'une collection de 74 souches de *Carnobacterium maltaromaticum* en lait entier UHT (ultra haute température). Chaque souche de *Carnobacterium maltaromaticum* a été co-cultivée avec *Listeria monocytogenes* EGDelux à différents taux d'ensemencement. La croissance de *Listeria monocytogenes* EGDe Lux est spécifiquement suivie par bioluminescence. Une croissance optimale de *Listeria monocytogenes* EGDe Lux est représentée en gris clair, une absence de détection de croissance de *Listeria monocytogenes* EGDe Lux est représentée en gris foncé, une croissance intermédiaire est représentée sous la forme d'un gradient de couleur allant du gris foncé au gris clair. Les lignes dans la partie « 1 » correspondent aux résultats obtenus pour un couple de souche *Carnobacterium maltaromaticum* / *Listeria monocytogenes* EGDe Lux à différents taux d'ensemencement. Les lignes dans la partie « 2 » sont les résultats obtenus pour les témoins positifs, correspondant à la co-culture de *Listeria monocytogenes* EGDe en présence de *Listeria monocytogenes* EGDe Lux. Les lignes dans la partie « 3 » sont les résultats des témoins négatifs, obtenue en présence de *Listeria monocytogenes* EGDe Lux.

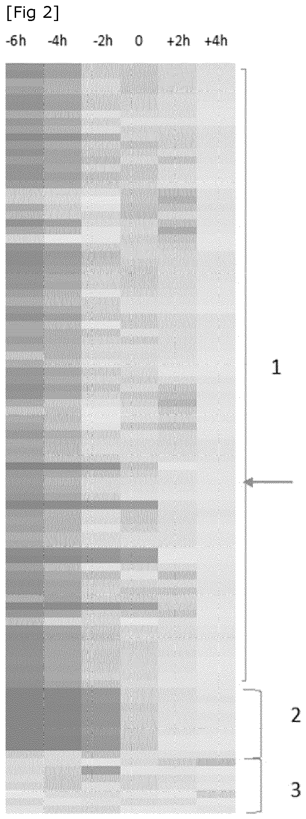

[Fig.2] Influence du temps d'ensemencement de *Carnobacterium maltaromaticum* sur les propriétés *anti-Listeria monocytogenes* d'une collection de 73 souches de *Carnobacterium maltaromaticum* en lait entier UHT. Chaque souche de *Carnobacterium maltaromaticum* est ensemencée à différents temps par rapport à *Listeria monocytogenes.* « -6h », « -4h », « -2h » signifient respectivement l'ensemencement de *Carnobacterium maltaroamticum* 6h, 4h, 2h avant d'ensemencer *Listeria monocytogenes.* « 0 » signifie l'ensemencement de *Carnobacterium maltaromaticum* en même temps que *Listeria monocytogenes. «* +2h », « +4h » signifient respectivement l'ensemencement de *Carnobacterium maltaromaticum* 2h, 4h après avoir ensemencé *Listeria monocytogenes.* Une croissance optimale de *Listeria monocytogenes* est représentée en gris clair, une absence de détection de croissance de *Listeria monocytogenes* est représentée en gris foncé, une croissance intermédiaire est représentée sous la forme d'un gradient de couleur allant du gris foncé au gris clair. Sur une ligne sont représentés les résultats obtenus pour un couple de souche *Carnobacterium maltaromaticum* / *Listeria monocytogenes* ensemencés avec différents décalages de temps. La flèche indique la ligne correspondant à la souche LMA28 qui est décrite dans l'art antérieur. Les lignes dans la partie « 1 » correspondent aux résultats obtenus pour un couple de souche *Carnobacterium maltaromaticum* / *Listeria monocytogenes* à différents taux d'ensemencement. Les lignes dans la partie « 2 » sont les résultats obtenus pour les témoins positifs. Les lignes dans la partie « 3 » sont les résultats des témoins négatifs.

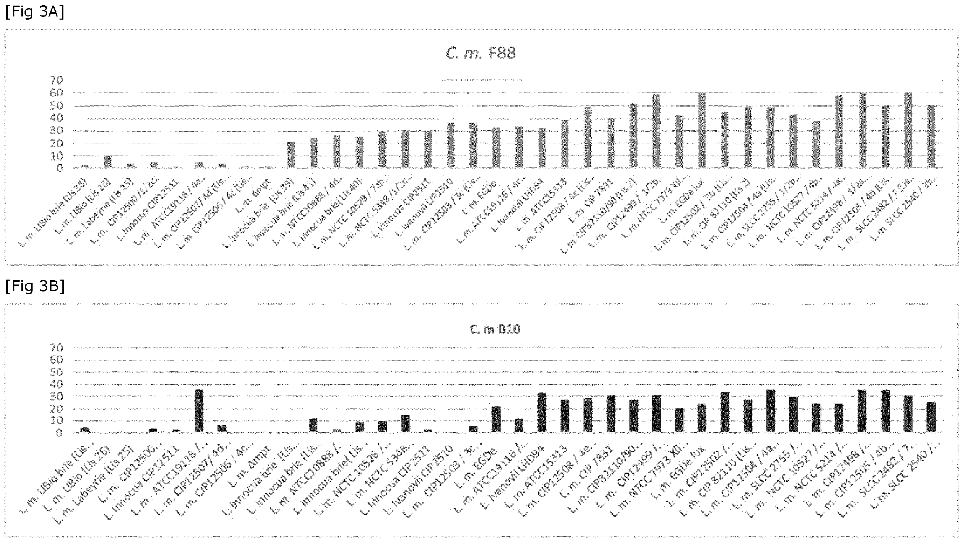

[Fig 3A] Activité inhibitrice de *Carnobacterium maltaromaticum* CNCM I-5242 (désignée dans les expériences présentées ci-après par F88) vis-à-vis de 38 souches de *Listeria* par mesure des zones d'inhibition (mm). L'axe des ordonnées représente les diamètres des zones d'inhibitions mesurés par la méthode de la double couche en boite de Petri.
[Fig 3B] Activité inhibitrice de *Carnobacterium maltaromaticum* CNCM I-5243 (désignée dans les expériences présentées ci-après par B10) vis-à-vis de 38 souches de *Listeria* par mesure des zones d'inhibition (mm). L'axe des ordonnées représente les diamètres des zones d'inhibitions mesurés par la méthode de la double couche en boite de Petri.

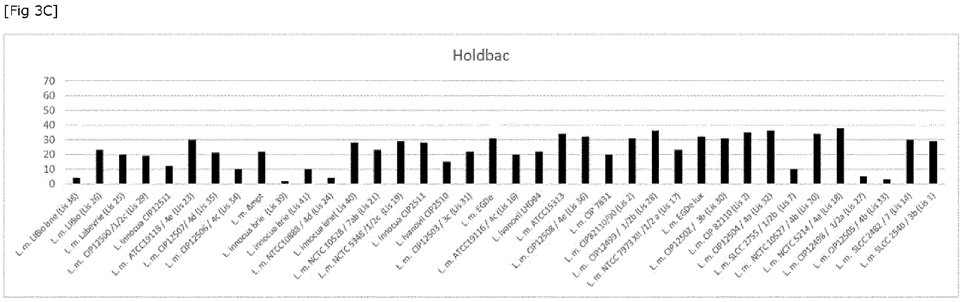

[Fig 3C] Activité inhibitrice de la souche de bioprotection *Lactobacillus plantarum* Holdbac vis-à-vis de 38 souches de *Listeria* par mesure des zones d'inhibition (mm). L'axe des ordonnées représente les diamètres des zones d'inhibitions mesurés par la méthode de la double couche en boite de Petri.

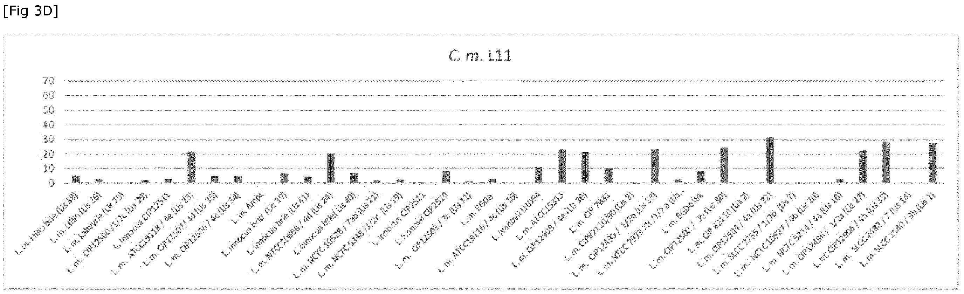

[Fig 3D] Activité inhibitrice de de *Carnobacterium maltaromaticum* L11 vis-à-vis de 38 souches de *Listeria* par mesure des zones d'inhibition (mm). L'axe des ordonnées représente les diamètres des zones d'inhibitions mesurés par la méthode de la double couche en boite de Petri.

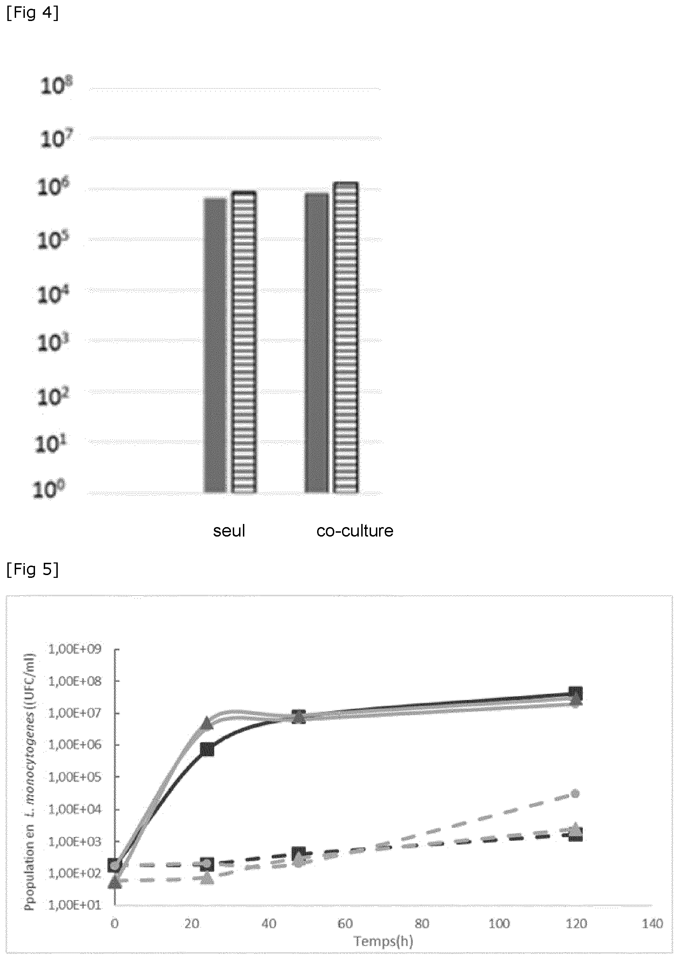

[Fig 4] Nivaux des populations de la souche CNCM I-5242 (désignée dans les expériences présentées ci-après par F88) de *Carnobacterium maltaromaticum* en culture seule ou en co-culture avec *Listeria monocytogenes* au temps initial (carré plein) et après 48 h de croissance (carré hachuré) à 14 °C. L'axe des ordonnées représente la population en *Carnobacterium maltaromaticum* (UFC.mL⁻¹).
[Fig 5] Evolution des populations au cours du temps, après croissance à 14 °C, de *Listeria monocytogenes* EGDe (triangle), *Listeria monocytogenes* Lis 1 (carré) et *Listeria monocytogenes* Lis 34 (rond) en culture seule (trait plein) ou en présence des souches de *Carnobacterium maltaromaticum Carnobacterium maltaromaticum C. m.* CNCM I-5242 (trait discontinu). L'axe des ordonnées représente la population en *Carnobacterium maltaromaticum* (UFC.mL⁻¹). L'axe des abscisses représente le temps d'incubation (heures).

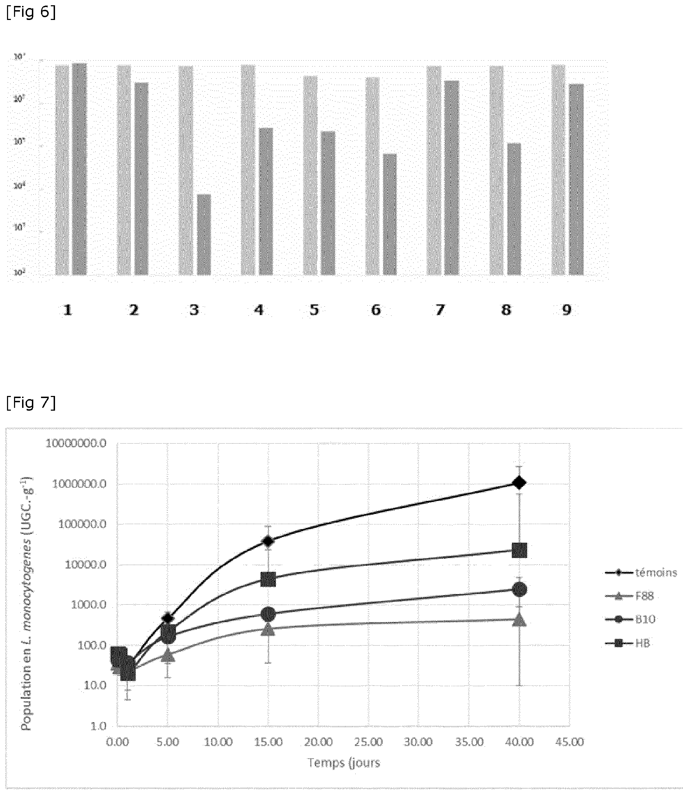

[Fig 6] Croissance de *Carnobacterium maltaromaticum C. m.* CNCM I-5242 en absence (1) et en présence des 8 ferments commerciaux au temps initial (gris clair) et après 24 h de co-culture (gris foncé), 1 : Témoin, 2 : Ferment Lc2a, 3 : Ferment Lc2b, 4 : Ferment Ic3, 5 : Ferment 5t, 6 : Ferment Lc-St, 7 : Ferment Lc-St-Lb, 8 : Ferment M.A-Lb, 9 : Ferment F.D.
[Fig 7] Comparaison de l'évolution au cours du temps des valeurs moyennes des populations en *Listeria monocytogenes* sur les 3 séries de fabrication de fromages à pâte molle dans les essais « témoins » avec *Listeria monocytogenes* seule sans culture protectrice (représenté par losange) ou en présence des différentes cultures protectrices *Carnobacterium maltaromaticum* CNCM I-5242 (désignée dans les expériences présentées ci-après par « F88 », représenté par triangle), *Carnobacterium maltaromaticum* CNCM I-5243 (désignée dans les expériences présentées ci-après par « B10 », représenté par point), et de la culture commercial Holdbac^{®} (désignée dans les expériences présentées ci-après par « HB », représenté par carré). L'axe des ordonnées représente la population en *Listeria monocytogenes* (UFC.g⁻¹). L'axe des abscisses représente le temps après inoculation (jours).

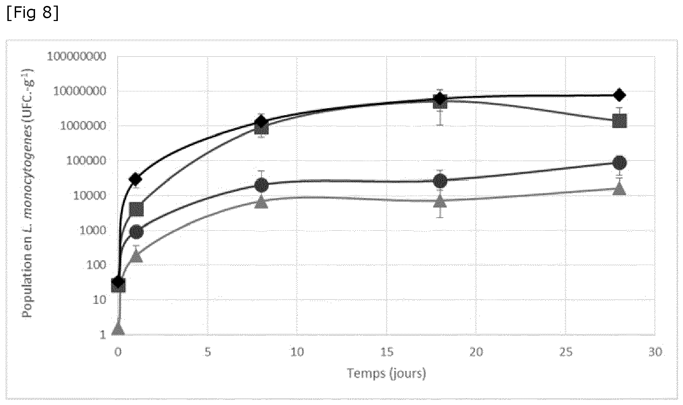

[Fig 8] Comparaison de l'évolution au cours du temps des valeurs moyennes des populations en *Listeria monocytogenes* lors d'une fabrication de type saint Nectaire dans les essais « témoins » (représenté par losange) ou en présence des cultures protectrices *Carnobacterium maltaromaticum* CNCM I-5242 (désignée dans les expériences présentées ci-après par « F88 », représenté par triangle) et *Carnobacterium maltaromaticum* CNCM I-5243 (désignée dans les expériences présentées ci-après par « B10 » représenté par point) et de la culture commercial Holdbac^{®} (désignée dans les expériences présentées ci-après par « HB », représenté par carré). L'axe des ordonnées représente la population en *Listeria monocytogenes* (UFC.g⁻¹). L'axe des abscisses représente le temps après inoculation (jours).

### Exemples

### Exemple 1 : Sélection des souches

Des souches de *Carnobacterium maltaromaticum* présentant une activité *anti-Listeria monocytogenes* sont d'abord identifiées en utilisant une seule souche de *Listeria monocytogenes.*

Ces souches ainsi identifiées constituent une collection pour le criblage suivent.

Ledit criblage est effectué par l'application simultanée des 3 critères expliqués ci-dessus :
- un faible taux d'ensemencement, à savoir inférieur à dés 10² UFC/mL de *Carnobacterium maltaromaticum* initial,
- une efficacité *anti-Listeria* même quand il y a un ensemencement tardif de *Carnobacterium maltaromaticum* par rapport à celui de *Listeria,* même en faible taux d'ensemencement, même en faible taux d'ensemencement
- un spectre d'activité vis-à-vis de diverses souches de *Listeria monocytogenes* et de *Listeria* large.

### Matériels et méthodes

### 1. Préparation de la pré-culture optimisée des souches de Carnobacterium maltaromaticum

Une collection de souches *Carnobacterium maltaromaticum* a été constituée au sein du laboratoire des Inventeurs par plusieurs campagnes d'isolement à partir de fromages, de sang humain et de lait cru. Le criblage de cette collection a permis d'identifier des souches aux propriétés *anti-Listeria monocytogenes.*

Une fraction d'un cryotube conservé à -80°C contenant une souche de *Carnobacterium maltaromaticum* à tester est prélevée stérilement à l'aide d'une öse stérile et repiquée dans 10 mL de bouillon TSB-YE, mis en incubation à 30 °C pendant 24 h. La culture bactérienne est centrifugée (3000 rpm, 10 min, 4 °C) puis lavée deux fois avec de l'eau physiologique stérile. Le culot bactérien est repris dans 10 mL de lait entier pasteurisé puis est introduit dans 100 mL de lait entier pasteurisé de façon à obtenir une population initiale proche de 10⁷ cellules.mL⁻¹. Le lait ensemencé est mis en incubation à 30 °C en bain-marie sous agitation. La croissance des souches de *Carnobacterium maltaromaticum* est suivie par numération sur milieu TSA-YE pendant 48 h. La pré-culture des souches permet l'obtention de grandes quantités de cellules viables, métaboliquement actives sans phase de latence.

### 2. Souches Listeria monocytogenes

Les souches de *Listeria monocytogenes* utilisées dans les expériences proviennent de collections publiques (American Type Culture Collection - USA (ATCC) ; Collection de l'Institut Pasteur - France (CIP) ; National Collection of Type culture - Great Britain (NCTC) ; Seeliger Listeria Culture Collection - Germany), de laboratoire de recherche (EGDe, EGDe Lux, Δmpt) et du laboratoire des Inventeurs. Les souches testées comptent 31 souches de *Listeria monocytogenes,* 2 souches de *Listeria ivanovii* et 5 souches de *Listeria innocua* (Tableau 1 ci-après). Les souches sont revivifiées à partir de cultures congelées à -80°C (Cryotubes de culture TSB-YE +15% de Glycérol) dans un bouillon de culture TSB-YE et incubées à leur température optimale de croissance, soit 30 °C, pendant 24 h.

La souche *Listeria monocytogenes* EGDe Lux est utilisée pour l'expérience de bioluminescence.

**Tableau 1 : Liste des souches de Listeria sélectionnées pour l'étude de l'activité anti-Listeria des souches de Carnobacterium maltaromaticum (American Type Culture Collection - USA (ATCC) ; Collection de l'institut Pasteur - France (CIP) ; National Collection of Type culture - Great Britain (NCTC) ; Seeliger Listeria Culture Collection - Germany)**

| [Tableau 1 ] | | | |
|---|---|---|---|
| **Noms** | **Origine / sérotype** | **Code** | **Année d'isolement** |
| *Listeria monocytogenes* | ATCC 19118 /4e | Lis 23 | 1995 |
| | ATCC 19116 / 4c | Lis 16 | 1995 |
| | ATCC 15313 | | - |
| | CIP 82110 / 90 | Lis 2 | 1994 |
| | CIP 12498/ ½a | Lis 27 | 1994 |
| | CIP12499/ ½b | Lis 28 | 1994 |
| | CIP 12500/ ½c | Lis 29 | 1994 |
| | CIP 12502/ 3b | Lis 30 | 1994 |
| | CIP 12503/ 3c | Lis 31 | 1994 |
| | CIP 12504/ 4a | Lis 32 | 1994 |
| | CIP 12505/ 4b | Lis 33 | 1994 |
| | CIP 12506/ 4c | Lis 34 | 1994 |
| | CIP 12507/ 4d | Lis 35 | 1994 |
| | CIP 12508/ 4e | Lis 36 | 1994 |
| | CIP 7831 | | - |
| | CIP 82110 | | - |
| | NTCC 7973 XII | Lis 17 | 1995 |
| | NCTC 5214/ 4a | Lis 18 | 1995 |
| | NCTC 5348 / ½c | Lis 19 | 1995 |
| | NITC 10527 / 4b | Lis 20 | 1995 |
| | NCTC 10528 / 7ab | Lis 21 | 1995 |
| | NTCC10888/4d | Lis 24 | 1995 |
| | SLCC 2755/ ½b | Lis 7 | 1995 |
| | SLCC 2540/3b | Lis1 | 1995 |
| | SLCC 2482/7 | Lis 14 | 1995 |
| | LIBio brie | Lis 38 | 1989 |
| | LIBio Labeyrie | Lis 25 | 1994 |
| | Lis LIBio | Lis 26 | |
| | Δ mpt* | | - |
| | EGDe | | - |
| | EGDe Lux | | - |
| *Listeria innocua* | CIP 12511 | | - |
| | CIP 2511 | | - |
| | LIBio brie | Lis 41 | 1989 |
| | LIBio brie | Lis 40 | 1989 |
| | LIBio brie | Lis 39 | 1989 |
| *Listeria ivanovii* | CIP 2510 | | - |
| | LH D94 | | - |
| | | | |

| | | | |
|---|---|---|---|
| *souche modifiée pour être résistante aux bactériocines de classe IIa, anti-*Listeria monocytogenes* | | | |

### 3. Souches témoins

Une souche *Lactobacillus plantarum* HOLDBAC^{®} connue pour son activité anti-*Listeria* et déjà commercialisée en tant que ferment de bio-préservation a été utilisée comme témoin afin de comparer l'activité anti-*Listeria* des souches de *Carnobacterium maltaromaticum* avec celles d'un ferment de bio-protection commercialisé.

La souche *Carnobacterium maltaromaticum* LMA28 décrite par Afzal et al. (Food Microbiology 36(2013) 223-230) a également utilisée dans certaines expériences comme témoin.

### 4.1 Activité anti-Listeria des souches de Carnobacterium maltaromaticum

L'activité *anti-Listeria* des souches de *Carnobacterium maltaromaticum* a été déterminée par la méthode de la double couche en boite de Petri contenant le milieu gélosé TSA-YE (Biomerieux, Marcy- l'Etoile, France). Une colonie de *Carnobacterium maltaromaticum* est ensemencée par piqure à la surface d'un milieu gélosé TSA-YE (Biomerieux, Marcy- l'Etoile, France) coulé en boite de Petri puis est incubée à 30 °C jusqu'à l'apparition d'une colonie (24 h). Les souches de *Listeria* sont repiquées dans du bouillon TSB-YE et incubées à 30°C pendant 24 h. Les souches de *Listeria monocytogenes* sont diluées au centième dans du milieu gélosé TSA-YE maintenu en surfusion à 50 °C, de façon à obtenir une concentration d'environ 10⁶ cellules/mL. Un volume de 15 mL de milieu gélosé est versé à la surface des géloses TSA-YE inoculées par les souches de *Carnobacterium maltaromaticum* (ou de *Listeria plantarum* Holdbac). Les boites de Petri sont ensuite incubées à 30°C pendant 24h. L'activité inhibitrice est évaluée en mesurant les diamètres des zones d'inhibition entourant chaque souche productive (en millimètres).

### 4.2 Activité anti-Listeria des souches de Carnobacterium maltaromaticum par test de bioluminescence

Les pré-cultures initiales en lait entier pasteurisé des différentes souches de *Carnobacterium maltaromaticum* sont préparées comme indiqués précédemment. La souche *Listeria monocytogenes* EGDe Lux est revivifiée à partir d'une culture congelée à -80°C (Cryotubes de culture TSB-YE +15% de Glycérol) dans un bouillon de culture TSB-YE et incubée à 30 °C pendant 24 h. A l'aide du robot (TECAN), des plaques de 96 puits sont remplies avec 160 µL de lait entier ou demi-écrémé et ensemencées avec 20 µL de la pré-culture de *Listeria monocytogenes* EGDe lux diluée au 1/100 0000^{éme}. Des dilutions en cascade au 1/10^{éme} des cultures de *Carnobacterium maltaromaticum* sont réalisées dans du lait entier ou demi-écrémé. Un volume de 20 µL des 4 premières dilutions est déposé dans les puits en suivant un plan de plaque prédéfini. Des essais avec des pré-cultures de 24 h de *Carnobacterium maltaromaticum* en milieu TSB-YE ainsi que des cultures de *Listeria monocytogenes* EGDe Lux cultivées en absence de souches de *Carnobacterium maltaromaticum* servent de témoins. Trois répétitions sont réalisées. La bioluminescence est mesurée grâce à un lecteur de microplaque Infinite 200 Pro équipé d'un module de luminescence (TECAN group Ltd. Mânnedorf, Switzerland). Les mesures sont réalisées sur des microplaques blanches opaques.

### Résultats

### Influence du taux d'ensemencement de Carnobacterium maltaromaticum

L'influence du taux d'ensemencement sur les propriétés *anti-Listeria monocytogenes* de *Carnobacterium maltaromaticum* est évaluée par luminescence. Chaque souche de *Carnobacterium maltaromaticum* a été co-cultivée avec *Listeria monocytogenes* à différents taux d'ensemencement. La croissance *de Listeria monocytogenes* est spécifiquement suivie par luminescence.

Une collection de souches de *Carnobacterium maltaromaticum* à cribler est analysée par cette méthode afin de sélectionner les souches qui présentent une activité *anti-Listeria monocytogenes* même en faible taux d'ensemencement.

Cette expérience montre qu'à haute concentration toutes les souches de *Carnobacterium maltaromaticum* sont capables d'inhiber *Listeria monocytogenes.* En revanche, à faible concentrations en *Carnobacterium maltaromaticum* et *Listeria. monocytogenes,* conditions plus représentatives des taux de contamination par *Listeria monocytogenes* dans les aliments, seules quelques souches de *Carnobacterium maltaromaticum,* y compris la souche CNCM I-5242 (désignée dans les expériences présentées ci-après par F88) et la souche CNCM I-5243 (désignée dans les expériences présentées ci-après par B10), inhibent encore *Listeria monocytogenes.* Le résultat est montré dans la figure 1.

### Décalage temporaire réduit de l'ensemencement de Carnobacterium maltaromaticum

L'influence du décalage d'ensemencement de *Carnobacterium maltaromaticum* sur les propriétés *anti-Listeria monocytogenes* est évaluée par luminescence.

Les carnobactéries ont été ensemencées soit 6h, 4h, 2h avant l'ensemencement de *Listeria monocytogenes,* ou en même temps que l'ensemencement de *Listeria monocytogenes,* soit 2h ou 4h après l'ensemencement de *Listeria monocytogenes.*

Le résultat montre que lorsque les souches de *Carnobacterium maltaromaticum* sont ensemencées 6h avant *Listeria monocytogenes,* de très nombreuses souches peuvent inhiber *Listeria monocytogenes.*

En revanche, plus le temps d'avance est réduit plus le nombre de souches capables d'inhiber *Listeria* diminue. Ainsi, lorsque les souches sont ensemencée en même temps (condition « 0 ») seules 4 souches, y compris la souche CNCM I-5242 (désignée dans les expériences présentées ci-après par F88) et la souche CNCM I-5243 (désignée dans les expériences présentées ci-après par B10), inhibent encore efficacement *Listeria monocytogenes* (figure 2).

La figure 2 montre que la souche LMA28 mentionnée dans l'art antérieur présente une activité inhibitrice assez faible aux conditions testées.

### Activité anti-Listeria de Carnobacterium maltaromaticum vis-à-vis différentes souches de Listeria

Afin de déterminer si une souche de *Carnobacterium maltaromaticum* présente un spectre d'activité *anti-Listeria* large, l'activité *anti-Listeria* de ladite souche a été évaluée vis-à-vis d'une collection de 31 souches de *Listeria monocytogenes,* 2 souches de *Listeria ivanovii* et 5 souches de *Listeria innocua* (Tableau 1 ci-dessus). Une souche *Lactobacillus plantarum* HOLDBAC^{®} connue pour son activité *anti-Listeria* et déjà commercialisée en tant que ferment de biopréservation a été utilisée comme témoin afin de comparer l'activité *anti-Listeria* des souches de *Carnobacterium maltaromaticum* avec celles d'un ferment de bioprotection commercialisé.

Les deux souches objet de la présente invention CNCM I-5242 et CNCM I-5243 présentent une forte activité *anti-Listeria* vis-à-vis de la plupart des souches de Listeria testées dans cette expérience. Pour certaines souches de *Listeria,* la souche CNCM I-5242 (désignée dans les expériences présentées ci-après par F88) de la présente invention montre même une meilleure activité inhibitrice par rapport à la souche *Lactobacillus plantarum* HOLDBAC^{®} (figure 3A, 3B, 3C).

La figure 3D montre comme contre-exemple le résultat obtenu pour une souche de *Carnobacterium maltaromaticum* désignée L11 qui est testé dans les mêmes conditions d'expérience. Alors que cette souche a été retenue dans un premier temps pour son activité inhibitrice de *Listeria monocytogenes* EGDe Lux dans le test par bioluminescence, cette souche ne montre pas une activité anti-*Listeria* suffisante vis-à-vis de la plupart des souches de *Listeria* testées dans cette expérience.

### Exemple 2 : Validation de l'activité anti-Listeria monocytogenes des souches de Carnobacterium maltaromaticum par co-culture en milieu lait

Il a été constaté que les souches de *Listeria monocytogenes* présentent des niveaux de sensibilité différents, visualisés par des diamètres d'inhibition variant de 1 à 64 mm. Il est nécessaire, en fromagerie, que la croissance de toutes les souches de *Listeria monocytogenes,* quelle que soit leur origine, soit inhibée. Lors des études précédentes, la concentration en *Listeria* utilisée afin de déterminer le spectre d'activité était élevée, proche de 10⁶ cellules.mL⁻¹. En fabrication fromagère les niveaux de contamination en *Listeria* sont faibles, inférieurs à 100 cellules.mL⁻¹. Les essais précédents ont été réalisés en milieu de laboratoire, alors que les souches inhibitrices doivent être efficaces en milieu lait.

Afin de tenir compte de tous ces facteurs, il est nécessaire :
- d'analyser l'efficacité inhibitrice des souches de *Carnobacterium maltaromaticum* sélectionnées vis-à-vis de la croissance de *Listeria monocytogenes* dans du lait, en présence de niveaux de contamination proches de ceux rencontrés dans les fromages ;
- de déterminer si les souches les moins sensibles de *Listeria monocytogenes* sont inhibées lorsque des niveaux d'ensemencement faibles en *Listeria* sont utilisés.

L'objectif de ces travaux est d'analyser l'efficacité inhibitrice des souches de *Carnobacterium maltaromaticum* sélectionnées vis-à-vis de la croissance de *Listeria monocytogenes* dans du lait, en présence de niveaux de contamination proches de ceux rencontrés dans les fromages et de déterminer si les souches les moins sensibles de *Listeria monocytogenes* sont inhibées lorsque des niveaux d'ensemencement faibles en *Listeria* sont utilisés. Les trois souches de *Listeria monocytogenes, Listeria monocytogenes* CIP 12506 (Lis 1), *Listeria monocytogenes* EGDe et *Listeria monocytogenes* SLCC 2540 (Lis 34), représentatives de 3 niveaux de sensibilité - respectivement fort, moyen et bas - sont utilisées pour ces études.

Matériels et Méthodes : La souche CNCM I-5242 (désignée dans les expériences présentées ci-après par F88) de *Carnobacterium maltaromaticum* est pré-cultivées 48 h dans du lait entier pasteurisé (cf. partie précédente). Les trois souches de *Listeria monocytogenes, Listeria monocytogenes* CIP 12506 (Lis 1), *Listeria monocytogenes* EGDe et *Listeria monocytogenes* SLCC 2540 (Lis 34) sont revivifiées à partir d'une culture congelée à -80°C (Cryotubes de culture TSB-YE +15% de Glycérol) dans un bouillon de culture TSB-YE et incubée à 30 °C pendant 24 h. Les culots bactériens sont ensuite repris dans 10 mL de lait entier pasteurisé après deux lavages dans de l'eau physiologique. Un volume de 1 mL de cultures de *Carnobacterium maltaromaticum* sert à ensemencer 100 mL de lait entier pasteurisé auquel est ajouté chacune des souches de *Listeria monocytogenes,* diluée de façon à obtenir une population initiale proche de 50 UFC.mL⁻¹. Des témoins de culture sont réalisés en absence de chacune des souches de *Listeria monocytogenes* et en absence de la souche de *Carnobacterium maltaromaticum.* Les populations en *Listeria monocytogenes* sont énumérées par comptage des colonies obtenues après suspension/dilution en Tryptone-Sel de l'échantillon et étalement sur milieu PALCAM incubée 24 h à 30°C. Les populations de *Carnobacterium maltaromaticum* sont dénombrées après 48 h selon la même technique en utilisant le milieu sélectif MCM (Medium for Carnobacterium Maltaromaticum) ; incubé 24 h à 25 °C (Edima et al., 2007 J. Food Microbiol., 67 :516-521). Les populations en *Listeria monocytogenes* sont déterminées pendant 120h après incubation à 14 °C (température de pré-maturation froide).

### Résultat

La présence des différentes souches de *Listeria monocytogenes* ne modifie pas la croissance de *Carnobacterium maltaromaticum* (figure 4). La population de *Carnobacterium maltaromaticum* reste stable lors des cultures à 14 °C après 48h. Après 120 h de culture la population en *Carnobacterium maltaromaticum* augmente d'environ 0,5 à 1 log lors des incubations à 14 °C.

A 14°C, les 3 souches de *Listeria monocytogenes* se développent sur lait atteignant des populations comprises entre 5×10⁵ et 5×10⁶ UFC.mL⁻¹ en 24h. Les niveaux des populations sont de l'ordre de 5×10⁷ UFC.mL⁻¹ après 120 h de croissance. En co-culture avec la souche *Carnobacterium maltaromaticum C. m* CNCM I-5242 (F88), la croissance des 3 souches de *Listeria monocytogenes* est inhibée pendant au moins 48h, en restant à des niveaux de populations identiques aux niveaux initiaux. Après 120 h, les niveaux des populations en *Listeria monocytogenes* sont 3 à 4 log inférieurs à ceux des témoins (figure 5).

### Exemple 3 : Etude de compatibilité des souches de Carnobacterium maltaromaticum avec les bactéries lactiques et les ferments d'acidification

L'utilisation d'un auxiliaire technologique ne doit pas avoir d'impact majeur sur la phase d'acidification laquelle est assurée par les bactéries lactiques qui entrent dans la composition des ferments d'acidification. De façon analogue, les bactéries lactiques ne doivent pas inhiber la croissance des cultures de *Carnobacterium maltaromaticum* afin qu'elles puissent continuer à se développer lors de la phase d'affinage du fromage.

Il est important d'étudier l'impact des bactéries lactiques sur la viabilité des souches de *Carnobacterium maltaromaticum* ainsi que l'impact de ces souches de *Carnobacterium maltaromaticum* sur les bactéries lactiques, et notamment sur les cinétiques d'acidification des ferments.

### Impact des ferments sur la croissance des souches de Carnobacterium maltaromaticum en co-culture

L'impact des ferments sur la viabilité des souches de *Carnobacterium maltaromaticum* a été analysé lors de co-culture.

Les ferments utilisés sont listés dans le tableau 2 ci-après.

**Tableau 2 : Références, composition et utilisation commerciale des différents ferments utilisés**

| [Tableau 2] | | | | | | | | |
|---|---|---|---|---|---|---|---|---|
| Ferments | Souches de bactéries lactiques | | | | | | | Utilisation |
| | *Lactococcus lactis* subsp. | | | *Sreptococcus thermophillus* | *Lactobacillus* | | *Leuconostoc* | |
| | *cremoris* | *lactis* | *lactis* biovar *diacetylactis* | | *helveticus* | *lactis* | | |
| Ferment Lc2a : Choozit^{®} MA 16 lyophilisé 25DCU (mésophile homofermentaire - Danisco) | X | X | | | | | | pâte molle, pâte cuite, pâte pressée, quark type, crème acide, fromage frais |
| Ferment Lc2b: Choozit^{®} MA 14 lyophilisé 50DCU (mésophile homofermentaire: Danisco) | X | X | | | | | | pâte molle, pâte cuite, pâte pressée, quark type, crème acide, fromage frais |
| Ferment Lc3 : PAL^{®} 122 D2U (mésophile hétérofermentaire - Standa) | X | X | X | | | | | pâte molle, pâte demi-dure, pâte cuite, crème acide, fromage frais |
| Ferment St : Choozit^{®} TA 60 lyophilisé 25DCU (ferment thermophile - Danisco) | | | | X | | | | pâte cuite, pâte pressée, asiago, quark type, crème acide |
| Ferment Le - St : Choozit^{®} MA 4001 lyophilisé 5DCU (mésophile homofermentaire - Danisco) | X | X | X | X | | | | pâte molle, pâte demi-dure, quark type, crème acide |
| Ferment Le - St - Lb: PAL^{®} cheese 114 D2U (mésophile et thermophile - Standa) | X | X | X | X | X | X | | pâte cuite, pâte pressée |
| Ferment FD : MSP lyophilisé 10U CHR Hansen | X | X | X | | | | X | pâte molle, bleu, pâte pressée |
| Ferment M.A. : ferment d'aromatisation | | | | | | | | |

Matériels et Méthodes : La souche *Carnobacterium maltaromaticum* CNCM I-5242 est pré-cultivée 48 h dans du lait entier pasteurisé (cf. partie précédente). Une quantité de 0,05 à 0,08 g de ferment lyophilisé est mis en culture dans 10 mL de lait entier pasteurisé pendant 24 h à 30 °C. Après croissance, des dilutions décimales, jusqu'à une dilution 10⁹, de cette culture sont réalisées dans du lait entier pasteurisé et mises en incubation 18 à 24 h à 30 °C. Le tube de culture précédent le dernier tube n'ayant pas entrainé la coagulation du lait est retenu pour ensemencer la co-culture. Un volume de 40 mL de lait entier pasteurisé est ensemencé avec un volume de 1 mL de pré-culture de *Carnobacterium maltaromaticum* et 0,1 mL de culture de ferment. Du lait ensemencé uniquement avec le ferment ou la culture de *Carnobacterium maltaromaticum* servent de témoin. L'ensemble est mis en incubation pendant 24 h à 30°C. Les populations bactériennes en *Carnobacterium maltaromaticum* sont déterminées après étalement de l'inoculum sur du milieu sélectif MCM incubé 48 h à 25°C selon la technique de suspension/dilution.

### Résultats :

Bien que la population finale de *Carnobacterium maltaromaticum* soit affectée par la présence des ferments (Figure 6), pour l'ensemble des souches de ferments testés, les cultures de *Carnobacterium maltaromaticum* restent cependant viables après un contact prolongé avec les ferments.

### Exemple 4 : Impact des cultures protectrices sur la croissance de Listeria monocytogenes lors de fabrications fromagères

### Préparation des cultures protectrices

Pour chaque souche bactérienne, à partir d'un cryotube conservé à -20 °C, 5 tubes de 10 mL de TSBYE sont préparés et notés avec le numéro de la souche et de a à e. Chacun de ces tubes, après centrifugation et lavage des cellules en eau physiologique stérile, sert à ensemencer un flacon de 100 mL de lait entier pasteurisé notés de a à e. Après croissance, chaque flacon a été congelé dans trois tubes Falcon contenant respectivement 50 mL, 50 mL et 10 mL de culture bactérienne. Ces tubes sont conservés à -20 °C. L'activité anti-bactérienne des différents lots de cultures protectrices vis-à-vis du mélange de souches de *Listeria monocytogenes* qui sont utilisées lors des essais de fabrication fromagère a été vérifiée.

### Protocole de fabrication de fromage de pâte molle

Trois séries de deux fabrications de 20 fromages à pâte molle sont réalisées simultanément. Une fabrication correspond à 10 cuves de 5L de lait (2 fromages de 250g/cuve) dont 8 cuves essais avec *Listeria monocytogenes* et les cultures protectrices fournies (dose indiquée) et 2 cuves témoins avec *Listeria monocytogenes* seule sans culture protectrice. Le lait utilisé est du lait pasteurisé et standardisé (MG/TP = 1, MG : matière gras, TP : taux protéique). Les ferments utilisés pour la technologie pâte molle sont le Flora Danica (FD, tableau 2) et le ferment RNA.

Les souches de *Carnobacterium maltaromaticum* (CNCM I-5242 (désignée dans les expériences présentées ci-après par F88) et CNCM I-5243(désignée dans les expériences présentées ci-après par B10)) sont conservées sous forme de cultures congelées prêtes à l'emploi pour chacune des souches à tester. Le mélange de 5 souches de *Listeria monocytogenes* est constitué des souches Scott A, CA2165 Fromage-CA Wisconsin E. Ryser, SN167 (souche isolée de fromage St-Nectaire), Lal65 (souche isolée de fromage Laguiole) et PL1547. Les pré-cultures des 5 souches (en individuel en BHI (brain heart infuction)) sont réalisées. Le mélange des 5 souches est dénombré sur milieu Palcam à 37°C, et conserver à - 20°C en aliquot de 1,5 ml. Un pré-essai a été validé pour vérifier le taux de croissance de *Listeria monocytogenes* selon cette technologie avec 2 taux d'inoculation de 50 et 100 ufc.mL⁻¹.

La culture protectrice commerciale Holdbac^{®} (désignée dans les expériences présentées ci-après par HB) a été utilisée en suivant les préconisations du fabricant pour comparer les résultats des cultures protectrices de *Carnobacterium maltaromaticum* à ceux obtenus avec cette culture commerciale.

### L'inoculation du mélange de Listeria monocytogenes à 50 ufc.mL⁻¹ et de Carnobacterium

*Carnobacterium maltaromaticum* (10⁶-10⁷ ufc.mL⁻¹) dans le lait est réalisé à J-1 (début maturation froide = T0). L'inoculation des ferments Flora Danica et RNA est réalisé à mi maturation froide (à T8h J-1) à un taux de 1%.

Une pulvérisation de *Geotricum* et *Pencillium candidum* en surface a lieu après salage.

Des échantillons sont prélevés à :
- T0 (J-1) = après inoculation des cultures protectrices et de Listeria = 30 ml du lait
- T8h (J-1) = 30 ml lait
- T20h (30) = fin maturation froide = 30 ml lait
- -T5j = ½ fromage : 25g pour dénombrement de *Listeria monocytogenes*
- -T15j = ½ fromage : 25g pour dénombrement de *Listeria monocytogenes*
- -T40j = ½ fromage : 25g pour dénombrement de *Listeria monocytogenes*

Le dénombrement de *Listeria monocytogenes* est effectué sur un mélange pâte et croûte.

Les pH des échantillons prélevés sont aussi mesurés.

### Résultats :

L'aspect de tous les fromages produits pendant l'expérience est correct tout au long de la fabrication. Tous les fromages ont le même aspect.

Les pH des fromages produits et les extraits secs obtenus sont corrects tout au long de la fabrication.

Le nombre de la population en *Listeria monocytogenes* dans les différents fromages produits est illustré dans la figure 7. Les résultats sont significativement différents entre les niveaux de population dans les essais témoins, en présence du ferment commercial Holdbac^{®} et en présence des 2 cultures protectrices de *Carnobacterium maltaromaticum.* Les écarts entre les niveaux de population apparaissent après 5 jours de fabrication et s'accentuent au cours de l'affinage. La population en *Listeria monocytogenes* est stabilisée dès 15 jours d'affinage en présence de la culture protectrice *Carnobacterium maltaromaticum* F88, alors qu'elle continue a augmenter en présence de *Carnobacterium maltaromaticum* B10 ou de la culture commerciale Holdbac^{®}. En revanche, les souches F88 et B10 montrent une meilleure activitié inhibitrice de *Listeria monocytogenes* par rapport à la culture protectrice commerciale Holdbac^{®}.

### Exemple 5 : Fabrications fromagères technologie pâte pressée non cuite type Saint Nectaire

### Protocole de fabrication de fromage à pâte pressée non cuite de type Saint Nectaire

Une fabrication correspond à 10 cuves de 5L de lait (1 fromage de 450g/cuve) ensemencées avec le mélange de souches de *Listeria monocytogenes.* Parmi celles-ci, deux séries de trois cuves sont ensemencées avec les cultures protectrices *Carnobacterium maltaromaticum* CNCM I-5242 (désignée dans les expériences présentées ci-après par F88) et *Carnobacterium maltaromaticum* CNCM I-5243 (désignée dans les expériences présentées ci-après par B10) et deux cuves avec la culture protectrice commerciale Holdbac^{®}. Deux cuves sans culture protectrice servent de témoin de croissance de *Listeria monocytogenes*

Le lait utilisé est du lait pasteurisé et standardisé (MG/TP=1). L'inoculation des ferments est réalisée à un taux de 1% dans le lait avant emprésurage et un mélange de bactéries pour l'affinage est pulvérisé en surface à J5 et J9.

Les cultures protectrices de *Carnobacterium maltaromaticum* sont des cultures congelées prêtes à l'emploi. L'inoculation est similaire à celle réalisée pour la technologie pâte molle, à savoir de 50 mL dans 5L de lait avant emprésurage. Le mélange de souches et les conditions d'ensemencement en *Listeria monocytogenes* sont similaires à ceux utilisés lors de la fabrication des pâtes molles.

L'affinage est réalisé pendant 28 jours.

Prélèvements et envois des échantillons :
- T0 lait avant emprésurage= inoculation des souches + *Listeria* = 30 ml du lait prélevé et congelé à -20°C.
- T1j = 1/4 fromage 25g pour dénombrement de *Listeria monocytogenes.*
- T8j = 1/4 fromage : 25g pour dénombrement de *Listeria monocytogenes*
- T18j = 1/4 fromage : 25g pour dénombrement de *Listeria monocytogenes*
- T28j = 1/4 fromage : 25g pour dénombrement de *Listeria monocytogenes.*

Le dénombrement de *Listeria monocytogenes* est effectué sur un mélange pâte et croûte.

Les pH des échantillons prélevés sont aussi mesurés.

### Résultat :

L'aspect de tous les fromages produits pendant l'expérence est correct tout au long de la fabrication. Tous les fromages ont le même aspect.

Les pH des fromages produits et les extraits secs obtenus sont corrects tout au long de la fabrication.

Le nombre de la population en *Listeria monocytogenes* dans les différents fromages produits est illustré dans la figure 8.

Les résultats de la fabrication de fromage de type Saint Nectaire sont similaires à ceux obtenus en technologie pâte molle :
- la population en *Listeria monocytogenes* est inférieure à celle des témoins en présence des 2 cultures protectrices de *Carnobacterium maltaromaticum* testées ;
- l'efficacité des 2 cultures protectrices de *Carnobacterium maltaromaticum* est supérieure à celle de la culture protectrice commerciale
- la culture protectrice *Carnobacterium maltaromaticum* F88 présente les meilleurs résultats d'inhibition.

## Revendications

1. Souche isolée de *Carnobacterium maltaromaticum* sélectionnée parmi *Carnobacterium maltaromaticum* déposée sous le numéro CNCM I-5242 et *Carnobacterium maltaromaticum* déposée sous le numéro CNCM I-5243.

2. Préparation bactérienne comprenant la souche CNCM I-5242 ou la souche CNCM I-5243.

3. Préparation bactérienne selon la revendication 2, ladite préparation comprenant en outre un ferment lactique et/ou un ferment d'affinage.

4. Préparation bactérienne selon la revendication 2 ou 3, **caractérisée en ce que** ladite préparation est un concentrât, un concentrât congelé, ou un lyophilisat.

5. Préparation bactérienne selon l'une quelconque des revendications 2-4, **caractérisée en ce que** ladite préparation est une culture protectrice de *Carnobacterium maltaromaticum.*

6. Utilisation d'une souche selon la revendication 1 ou d'une préparation bactérienne selon l'une quelconque des revendications 2 à 5 dans la préparation d'un produit alimentaire.

7. Utilisation selon la revendication 6, pour inhiber la croissance des bactéries du genre *Listeria* dans un produit alimentaire.

8. Utilisation selon la revendication 6 ou 7, **caractérisée en ce que** ledit produit alimentaire est un produit laitier fermenté, notamment les fromages, les laits fermentés.

9. Procédé de préparation d'un fromage, ledit procédé comprenant :
- l'ensemencement par au moins une souche de *Carnobacterium maltaromaticum* selon la revendication 1 ou une préparation bactérienne selon les revendications 2-5, dans le lait ou pendant la transformation du lait.

10. Procédé de préparation d'un fromage selon la revendication 9, **caractérisé en ce que** l'ensemencement par des bactéries *Carnobacterium maltaromaticum* est de 10² UFC.mL⁻¹ à 10¹¹ UFC.mL⁻¹, notamment de 10⁶ à 10⁷ UFC.mL⁻¹, par rapport au volume du lait.

11. Procédé pour réduire la contamination microbienne lors du processus de fabrication d'un produit laitier fermenté **caractérisé en ce que** qu'il comprend l'addition d'une quantité de 10² UFC.mL⁻¹ à 10¹¹ UFC.mL⁻¹, notamment de 10⁶ à 10⁷ UFC.mL⁻¹, par rapport au volume du produit laitier, de *Carnobacterium maltaromaticum* selon la revendication 1 ou une préparation bactérienne selon les revendications 2-5.

## Patentansprüche

1. Isolierter Stamm von *Carnobacterium maltaromaticum,* ausgewählt aus *Carnobacterium maltaromaticum* hinterlegt unter der Nummer CNCM I-5242 und *Carnobacterium maltaromaticum* hinterlegt unter der Nummer CNCM I-5243.

2. Bakterienpräparat, umfassend den Stamm CNCM I-5242 oder den Stamm CNCM I-5243.

3. Bakterienpräparat gemäß Anspruch 2, wobei das Präparat weiterhin einen Milchsäurekultur und/oder eine Reifekultur umfasst.

4. Bakterienpräparat gemäß Anspruch 2 oder 3, **dadurch gekennzeichnet, dass** das Präparat ein Konzentrat, ein Tiefkühlkonzentrat oder ein Lyophilisat ist.

5. Bakterienpräparat gemäß einem der Ansprüche 2-4, **dadurch gekennzeichnet, dass** das Präparat eine Schutzkultur von *Carnobacterium maltaromaticum* ist.

6. Verwendung eines Stammes gemäß Anspruch 1 oder eines Bakterienpräparats gemäß einem der Ansprüche 2 bis 5 bei der Herstellung eines Lebensmittels.

7. Verwendung gemäß Anspruch 6 zur Hemmung des Wachstums der Bakterien der Gattung *Listeria* in einem Lebensmittel.

8. Verwendung gemäß Anspruch 6 oder 7, **dadurch gekennzeichnet, dass** es sich bei dem Lebensmittel um ein fermentiertes Milchprodukt, insbesondere Käse oder fermentierte Milch, handelt.

9. Verfahren zur Herstellung eines Käses, wobei das Verfahren umfasst:
- Impfung von Milch mit wenigstens einem Stamm von *Carnobacterium maltaromaticum* gemäß Anspruch 1 oder einem Bakterienpräparat gemäß den Ansprüchen 2-5 von Milch oder während der Umwandlung der Milch.

10. Verfahren zur Herstellung eines Käses gemäß Anspruch 9, **dadurch gekennzeichnet, dass** die Impfung mit *Carnobacterium-maltaromaticum-*Bakterien mit 10² KBE/ml bis 10¹¹ KBE/ml, insbesondere 10⁶ bis 10⁷ KBE/ml, erfolgt, bezogen auf das Volumen der Milch.

11. Verfahren zum Senken der mikrobiellen Kontamination beim Vorgang der Herstellung eines fermentierten Milchprodukts, **dadurch gekennzeichnet, dass** es die Zugabe einer Menge von 10² KBE/ml bis 10¹¹ KBE/ml, insbesondere 10⁶ bis 10⁷ KBE/ml, bezogen auf das Volumen des Milchprodukts, von *Carnobacterium maltaromaticum* gemäß Anspruch 1 oder eines Bakterienpräparats gemäß den Ansprüchen 2-5 umfasst.

## Claims

1. Isolated strain of *Carnobacterium maltaromaticum* selected from *Carnobacterium maltaromaticum,* deposited under the number CNCM I-5242 and *Carnobacterium maltaromaticum,* deposited under the number CNCM I-5243.

2. Bacterial preparation comprising the strain CNCM I-5242 or the strain CNCM I-5243.

3. The bacterial preparation according to claim 2, said preparation further comprising a lactic fermenting agent and/or a ripening fermenting agent.

4. The bacterial preparation according to claim 2 or 3, **characterized in that** said preparation is a concentrate, a frozen concentrate, or a lyophilizate.

5. The bacterial preparation according to any one of claims 2-4, **characterized in that** said preparation is a protective culture of *Carnobacterium maltaromaticum.*

6. Use of a strain according to claim 1 or of a bacterial preparation according to any one of claims 2 to 5 in the preparation of a food product.

7. Use according to claim 6, in order to inhibit the growth of bacteria of the genus *Listeria* in a food product.

8. Use according to claim 6 or 7, **characterized in that** said food product is a fermented milk product, in particular cheeses, fermented milks.

9. Method for the preparation of a cheese, said method comprising:
- inoculation by at least one strain of *Carnobacterium maltaromaticum* according to claim 1 or a bacterial preparation according to claims 2-5, into milk or during the transformation of milk.

10. Method for the preparation of a cheese according to claim 9, **characterized in that** the inoculation with the *Carnobacterium maltaromaticum* bacteria is from 10² CFU.mL⁻¹ to 10¹¹ CFU.mL⁻¹, in particular from 10⁶ to 10⁷ CFU.mL⁻¹, with respect to the volume of milk.

11. Method for reducing microbial contamination during a process for the production of a fermented milk product, **characterized in that** it comprises adding a quantity of 10² CFU.mL⁻¹ to 10¹¹ CFU.mL⁻¹, in particular from 10⁶ to 10⁷ CFU.mL⁻¹, with respect to the volume of the milk product, of *Carnobacterium maltaromaticum* according to claim 1 or a bacterial preparation according to claims 2-5.
